# EUROPEAN PATENT SPECIFICATION

(11) **EP 3 836 922 B1**
(45) Date of publication and mention of the grant of the patent: **07.08.2024**
(21) Application number: 19849456.9
(22) Date of filing: 15.08.2019
(51) Int. Cl.: A61K 31/428, A61P 25/22, A61P 25/24, A61K 9/20

(54) **USE OF RILUZOLE ORAL DISINTIGRATING TABLETS FOR TREATING DISEASES**
VERWENDUNG VON RILUZOL-ORALEN DESINFEKTIONSMITTELTABLETTEN ZUR BEHANDLUNG VON KRANKHEITEN
UTILISATION DE COMPRIMÉS ORODISPERSIBLES À BASE DE RILUZOLE POUR LE TRAITEMENT DE MALADIES

(30) Priority: 16.08.2018 US 201862764864 P
(43) Date of publication of application: 23.06.2021
(73) Proprietor: Biohaven Therapeutics Ltd., New Haven, CT 06510 (US)
(72) Inventor: CORIC, Vladimir, New Haven, Connecticut 06510 (US)
(74) Representative: Mewburn Ellis LLP
(86) International application number: PCT/US2019/046709
(87) International publication number: WO 2020/037152

(56) References cited:
- WO-A1-2016/081466
- WO-A1-2016/140879
- CA-A1- 2 507 551
- US-A1- 2008 260 823
- US-A1- 2017 360 775
- US-A1- 2018 037 557
- US-A1- 2018 214 395
- US-B2- 9 434 704
- WILLIAMS K A ET AL: "Riluzole reduces anxiety-related behaviors in mice", SOCIETY FOR NEUROSCIENCE ABSTRACT VIEWER AND ITINERARY PLANNER, vol. 39, 2009, & 39TH ANNUAL MEETING OF THE SOCIETY-FOR-NEUROSCIENCE; CHICAGO, IL, USA; OCTOBER 17 -21, 2009, XP002806037
- MATHEW SANJAY J ET AL: "Open-label trial of riluzole in generalized anxiety disorder", AMERICAN JOURNAL OF PSYCHIATRY, vol. 162, no. 12, December 2005 (2005-12-01), pages 2379 - 2381, XP002806038, ISSN: 0002-953X
- ANONYMOUS: "Biohaven Announces Positive Results From Bioequivalence Study With Sublingual BHV-0223 Zydis Orally Dissolving Tablet", 12 January 2018 (2018-01-12), XP002806039, Retrieved from the Internet <URL:https://www.pharmasalmanac.com/articles/biohaven-announces-positive-results-from-bioequivalence-study-with-sublingual-bhv-0223-zydis-orally-dissolving-tablet> [retrieved on 20220323]
- ANONYMOUS: "Biohaven Initiates Expanded Access Program For Sublingual BHV-0223 Zydis", 10 May 2018 (2018-05-10), XP002806040, Retrieved from the Internet <URL:https://www.drugdiscoverytrends.com/biohaven-initiates-expandes-access-program-for-sublingual-bhv-0223-zydis/> [retrieved on 20220323]
- ANONYMOUS: "Riluzole", 23 March 2022 (2022-03-23), XP002806041, Retrieved from the Internet <URL:https://go.drugbank.com/drugs/DB00740> [retrieved on 20220324]
- HAESSLER, F ET AL.: "Characterization, treatment patterns, and patient-related outcomes of patients with Fragile X syndrome in Germany : final results of the observational EXPLAIN-FXS study", BMC PSYCHIATRY, vol. 16, no. 1, 10 September 2016 (2016-09-10), pages 318, XP055686624

## Description

This application claims priority to and the benefit of U.S. Provisional Application No. 62/764,864 filed on August 16, 2018.

### BACKGROUND OF THE INVENTION

### FIELD OF INVENTION

The present invention relates to a sublingual pharmaceutical composition for use in a method of treating an anxiety disorder and a kit thereof.

### BRIEF DESCRIPTION OF RELATED ART

Glutamate is a predominant excitatory neurotransmitter responsible for regulating signaling in normal brain function. While research on glutamate signaling has been primarily focused on the central nervous system (CNS), other investigations have highlighted their functional role in peripheral tissues. See, *e.g*., Skerry T, Genever P, Glutamate signaling in non-neuronal tissues. Trends Pharmacol. Sci. 2001, 22:174-181 and Frati C, Marchese C, Fisichella G, Copani A, Nasca MR, Storto M, Nicoletti F, Expression of functional mGlu5 metabotropic glutamate receptors in humanmelanocytes. J. Cell. Physiol. 2000, 183:364-372.

Glutamate can exert its signaling abilities by acting on glutamate receptors, which are located on the cell surface. Glutamate receptors exist as either ionotropic receptors (iGluRs) or metabotropic glutamate receptors (mGluRs). iGluRs are ligand-gated ion channels, which include *N*-methyl-d-aspartate (NMDA) receptors and non-NMDA receptors [*α*-amino-3-hydroxy-5-methyl-4-isoxazolepropionic acid (AMPA) receptors] (iGluR1-4) and kainite (KA) subfamilies (iGluR5-7, KA1, and KA2). mGluRs are domain receptors that mediate their signal by coupling to guanosine triphosphate (GTP)-binding proteins (G-proteins) and stimulate second messengers such as inositol 1,4,5-triphosphate (IP3), diacylglycerol (DAG), and cyclic adenosine monophosphate (cAMP). Various mGluR subtypes have been identified and grouped according to their sequence homology, pharmacologic response, and intracellular second messengers. Upon binding of the ligand, Group I receptors, which are comprised of mGluR1 and mGluR5, couple via G_{q} to phospholipase C (PLC) leading to the formation of IP3 and DAG. Group II comprises mGluR2 and mGluR3, and Group III comprises mGluR4, mGluR6, mGluR7, and mGluR8. Both Group II and III are negatively coupled via G_{i/o} to adenyl cyclase leading to cAMP formation. See, *e.g.,* Teh J, Chen S, Metabotrobic glutamate receptors and cancerous growth, WIREs Membr. Transp. Signal. 2012, 1:211-220; doi: 10.1002/wmts.21, 2011 WILEY-VCH Verlag GmbH & Co. KGaA, Weinheim. Volume 1, March/April 2012.

Glutamate can also be transported. Glutamate transporters have been cloned from the mammalian central nervous system. Two are expressed predominantly in glia [glial glutamate and aspartate transporter (GLAST) and glial glutamate transporter (GLT)] and three in neurons [EAAC1, excitatory amino acid transporter (EAAT)4 and EAAT5]. See, *e.g.,* Seal, R, Amara, S, (1999) Excitatory amino acid transporters: a family in flux. Annu. Rev. Pharmacol. Toxicol. 39: 431-456. Further information concerning glutamate transport can be found in the literature. See, *e.g.,* Meldrum B, Glutamate as a Neurotransmitter in the Brain: Review of Physiology and Pathology, J. Nutr. 130:1007S-1015S, 2000.

Glutamate can also be metabolized. Glutamate metabolism reactions can be catalyzed by enzymes that are regulated by activators and inhibitors. For instance, conversion of L-glutamate to N-acetyl L-glutamate in presence of N-acetylglutamate synthase (NAGS) is activated by L-arginine and inhibited by succinate, coenzyme A, N-acetyl-L-aspartate and N-acetyl-L-glutamate. See, *e.g*., Shigesada K, Tatibana M, N-acetylglutamate synthetase from rat-liver mitochondria. Partial purification and catalytic properties. Eur. J. Biochem. 1978; 84:285-291; doi: 10.1111/j.14321033.1978. tb12167.x. Similarly, glutamine to glutamate conversion can be catalyzed by enzymes, which include glutaminase (GLS/GLS2), phosphoribosyl pyrophosphate amidotransferase (PPAT) and glutamine-fructose-6-phosphate transaminase (GFPT1 and GFPT2). See, *e.g*., Holmes E, Wyngaarden J, Kelley W, Human glutamine phosphoribosylpyrophosphate amidotransferase. Two molecular forms interconvertible by purine ribonucleotides and phosphoribosylpyrophosphate. J. Biol. Chem. 1973;248:6035-6040, and Hu C, et al. Molecular enzymology of mammalian Delta 1-pyrroline-5-carboxylate synthase. Alternative Splice donor Utilization Generates Isoforms with Different Sensitivity to Ornithine Inhibition. J. Biol. Chem. 1999; 274:6754-6762; doi: 10.1074/jbc.274.10.6754.

Glutamine, which serves as a precursor of glutamate is known to protect the body from nutrient depletion, oxidative stress and tumor stress. See, *e.g.*, Shanware N, et al., Glutamine: pleiotropic roles in tumor growth and stress resistance. J. Mol. Med. (Berl.) 2011;89:229-236; doi: 10.1007/s0010901107319. Reports have shown that ammonia released from glutamine by the action of glutaminases regulates autophagy in cancer cells through a process known as glutaminolysis. See, *e.g.,* Eng C, et al., (2010) Ammonia derived from glutaminolysis is a diffusible regulator of autophagy. Sci. Signal. 3:ra31. In cancer cells, glutaminolysis may serve as a fuel for cell growth and proliferation through the synthesis of fatty acids, nucleotides and amino acids. See, *e.g*., Benjamin D, et al., Global profiling strategies for mapping dysregulated metabolic pathways in cancer. Cell Metab. 2012;16:565-577; doi: 10.1016/j.cmet.2012.09.013. Expression of glutaminase may be regulated by the transcription factor, c-Myc, which in turn regulates cell proliferation and cell death in human prostate cancer cells. See, *e.g.,* Gao P, et al., c-Myc suppression of miR23a/b enhances mitochondrial glutaminase expression and glutamine metabolism. Nature 2009;458:762-765; doi: 10.1038/nature07823. In brain tumors such as gliomas, it has been shown that glioma cells may release excess glutamate into the extracellular space resulting in tumor-related epilepsy or seizures. See, *e.g.,* Simon M, von Lehe M, Glioma-related seizures: glutamate is the key. Nαt Med. 2011;17:1190-1191; doi: 10.1038/nm.2510. There are also suggestions that glutamate release promotes cell proliferation, cell invasion and tumor necrosis in glioblastoma. See, *e.g.,* Schunemann D, et al., Glutamate promotes cell growth by EGFR signaling on U87MG human glioblastoma cell line. Pathol. Oncol. Res. 2010;16:285-293; doi: 10.1007/s1225300992234. Further information concerning glutamate and glutamine metabolism can be found in the literature. See, *e.g.,* Yelamanchi S., et al., A pathway map of glutamate metabolism, J. Cell. Commun. Signal. 2016 Mar: 10(1):69-76; doi10.1007/s12079-015-0315-5, and Chen L and Hengmin C, Targeting Glutamine Induces Apoptosis: A Cancer Therapy Approach, Int. J. Mol. Sci. 2015, 16, 22830-22855; doi:10.3390/ijms160922830.

Anxiety disorders are often debilitating chronic conditions, which can be present from an early age or begin suddenly after a triggering event. They are prone to flare up at times of high stress. Anxiety disorders include panic disorder, agoraphobia, social anxiety disorder (also known as social phobia, or SAD), specific phobia, or simple phobia, generalized anxiety disorder, obsessive-compulsive disorder, and post-trau-matic stress disorder. Social anxiety disorder is a marked and persistent fear of social situations, causing impairment and distress, which can impair school, work and social functioning. SAD affects approximately 12% of Americans. Roughly one-third to one-half of patients with SAD do not experience significant clinical benefit from current treatments, including selective serotonin reuptake inhibitors.

Alzheimer's disease is a progressive, fatal neurodegenerative dementia. It accounts for up to 80% of dementias. According to the Alzheimer's Association, in 2016 there were approximately 5.5 million people in the United States with the disease, and that number is expected to escalate rapidly in the coming years as the population ages. Reduced glutamate uptake transporters have been reported in postmortem brain tissue of individuals with Alzheimer's disease and the level of glutamate transporter reduction correlates with cognitive impairment as well as markers of synaptic density and neurodegeneration. Patients with AD may also lose memory as part of their condition.

The emotional and financial burden of AD to patients, family members, and society is enormous, and is predicted to grow exponentially as the median population age increases. The potential to preserve, or even improve, cognition in adults at high risk of cognitive decline due to AD clearly has important implications, not only for the affected individual, but also for the support system that bears the social and financial burdens of long-term caregiving.

There are medications currently approved for symptomatic treatment of AD, but they have small effect sizes and generally limited clinical benefits. An urgent need exists to find effective treatments for AD that can arrest or reverse the disease before its advanced stages. Therapeutic strategies aimed at restoring synaptic and extrasynaptic glutamate levels, offer potential therapeutic benefit in AD, in cognition, as well as in the neuroprotection of synapses, conferring the potential for disease modification. The significance of clinical research directed at this preclinically validated synaptic target cannot be overstated, given the lack of therapeutic progress in symptomatic and disease-modifying treatments since 2003.

The FDA originally approved riluzole (RILUTEK^{®}) 50 mg twice-a-day (NDA #20-599) for the treatment of patients with amyotrophic lateral sclerosis (ALS). Riluzole is only indicated for ALS and has a number of non-desirable attributes that have limited its clinical use.

Riluzole tablets have 60% bioavailability, attributed to high first-pass metabolism in the liver. This is thought to be related to metabolism by the heterogeneously expressed CYP1A2 enzyme, which also accounts for the high PK variability associated with riluzole (Carlsson, 2000; Pittenger, 2015a, 2015b). In addition, riluzole is associated with reduced exposure when taken with meals (*i.e.,* a negative food effect), resulting in the guidance to take riluzole within a three hour fast (one hour before or two hours after a meal).

Riluzole is also dosed twice a day, has dose-dependent effects on liver function tests and the drug substance itself has other intrinsic limitations including: very low solubility in water, poor oral palatability, pH dependent chemical stability, and intense oral numbness if administered directly to the oral mucosa.

Recently, riluzole has been shown to have other clinical benefits. For example, orally administered riluzole dosed twice a day at a total dose of 100 mg per day may relieve or treat neuropsychiatric symptoms and disorders, such as mood, anxiety disorder, refractory depression, obsessive-compulsive anxiety and the like. See, *e*.*g*., Riluzole Augmentation in Treatment-refractory Obsessive-compulsive Disorder, Yale University (2016) Retrieved from https://clinicaltrials.gov/ct2 (Identification No. NCT00523718). Also, there is some indication that riluzole may have anti-cancer effects. See, *e*.*g*., Riluzole in Treating Patients With Stage III or Stage IV Melanoma That Cannot Be Removed by Surgery, Rutgers University (2013) Retrieved from https://clinicaltrials.gov/ct2 (Identification No. NCT00866840).

Accordingly, new compounds, pharmaceutical compositions and methods are desired for the treatment of anxiety disorders, including without limitation SAD, Alzheimer's Disease, and memory enhancement which may provide benefits for patients afflicted with the diseases or conditions.

### SUMMARY OF THE INVENTION

The present invention is defined in the appended claims. It is directed to methods of treating social anxiety disorder in patients in need thereof, comprising administering to the patient a pharmaceutical composition comprising a therapeutically effective amount of riluzole, or a pharmaceutically acceptable salt thereof, in the form of an oral solid molded fast-dispersing dosage form.

In one aspect of the invention, there is provided a sublingual pharmaceutical composition for use in a method of treating social anxiety disorder (SAD), the composition comprising riluzole, or a pharmaceutically acceptable salt or prodrug thereof, in the form of an oral solid molded fast-dispersing dosage form in order to provide a reduction of at least 10 VAS points as compared to administration of a placebo; wherein riluzole prodrugs are according to the following formula: 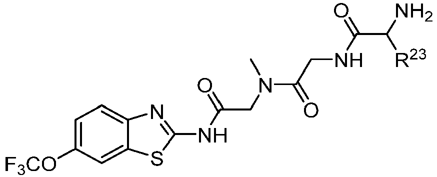 and pharmaceutically acceptable salts thereof, wherein:
R₂₃ is selected from the group consisting H, CH₃, CH₂CH₃, CH₂CH₂CH₃, CH₂CCH, CH(CH₃)₂, CH₂CH(CH₃)₂, CH(CH₃)CH₂CH₃, CH₂OH, CH₂OCH₂Ph, CH₂CH₂OCH₂Ph, CH(OH)CH₃, CH₂Ph, CH₂(cyclohexyl), CH₂(4-OH-Ph), (CH₂)₄NH₂, (CH₂)₃NHC(NH₂)NH, CH₂(3-indole), CH₂(5-imidazole), CH₂CO₂H, CH₂CH₂CO₂H, CH₂CONH₂, and CH₂CH₂CONH₂.

According to the invention, the dosage of riluzole in the oral solid molded fast dispersing tablet is from 20 to 50 mg.

In one aspect, the dosage of riluzole in the oral solid molded fast dispersing tablet is about 35 mg.

In one aspect, administration provides a reduction of at least 12 VAS points when tested according to the procedure set forth in Example 1.

In one aspect, administration provides a reduction of at least 14 VAS points when tested according to the procedure set forth in Example 1.

In one aspect, administration provides a reduction of from about 10 to 25 VAS points when tested according to the procedure set forth in Example 1.

In one aspect, administration provides a mean VAS Score of from about 49 to 60 when tested according to the procedure set forth in Example 1.

In one aspect, administration provides a mean VAS Score of from about 52 to 58 when tested according to the procedure set forth in Example 1.

In one aspect, the disease is SAD.

In one aspect, there is an enhancement in memory of the patient.

In one aspect, the oral solid molded fast-dispersing dosage form comprises from about 50-70 weight% riluzole, pharmaceutically acceptable salt or prodrug thereof, about 10-30 weight% fish gelatin, about 10-20 weight% of a filler, and 0.1-5.0 weight% of a flavorant.

In one aspect, the filler is mannitol.

In one aspect, the riluzole prodrug has the following formula:

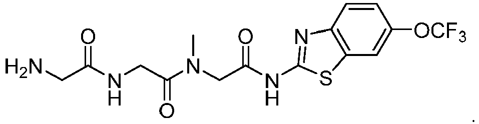

In one aspect of the invention, there is provided a kit for use in a method of treating an anxiety disorder, the kit comprising:
(a) a sublingual pharmaceutical composition according to an aspect of the invention; and
(b) instructions for administering the pharmaceutical composition.

### BRIEF DESCRIPTION OF THE DRAWINGS

These and/or other aspects will become apparent and more readily appreciated from the following description of the embodiments, taken in conjunction with the accompanying drawings in which:
FIG. 1 illustrates primary endpoints of mean anxiety rating as measured by the Visual Analogue Scale at baseline and during an anxiety-provoking, impromptu speech task;
FIG. 2 illustrates primary endpoints of self-rated anxiety rating as measured by the Visual Analogue Scale at baseline and during an anxiety-provoking, impromptu speech task; and
FIG. 3 illustrates primary endpoints of the immediate and delayed word recall test.

### DETAILED DESCRIPTION OF THE INVENTION

The following detailed description is provided to aid those skilled in the art in practicing the present invention. Those of ordinary skill in the art may make modifications and variations in the embodiments described herein. Unless otherwise defined, all technical and scientific terms used herein have the same meaning as commonly understood by one of ordinary skill in the art to which this disclosure belongs. The terminology used in the description is for describing particular embodiments only and is not intended to be limiting.

As used in this application, except as otherwise expressly provided herein, each of the following terms shall have the meaning set forth below. Additional definitions are set forth throughout the application. In instances where a term is not specifically defined herein, that term is given an art-recognized meaning by those of ordinary skill applying that term in context to its use in describing the present invention.

The articles "a" and "an" refer to one or to more than one (*i.e.,* to at least one) of the grammatical object of the article unless the context clearly indicates otherwise. By way of example, "an element" means one element or more than one element.

The term "about" refers to a value or composition that is within an acceptable error range for the particular value or composition as determined by one of ordinary skill in the art, which will depend in part on how the value or composition is measured or determined, *i.e.,* the limitations of the measurement system. For example, "about" can mean within 1 or more than 1 standard deviation per the practice in the art. Alternatively, "about" can mean a range of up to 10% or 20% *(i.e.,* ±10% or ±20%). For example, about 3 mg can include any number between 2.7 mg and 3.3 mg (for 10%) or between 2.4 mg and 3.6 mg (for 20%). Furthermore, particularly with respect to biological systems or processes, the terms can mean up to an order of magnitude or up to 5-fold of a value. When particular values or compositions are provided in the application and claims, unless otherwise stated, the meaning of "about" should be assumed to be within an acceptable error range for that particular value or composition.

The term "administering" refers to the physical introduction of a composition comprising a therapeutic agent to a subject, using any of the various methods and delivery systems known to those skilled in the art. For example, routes of administration for riluzole can include bucal, intranasal, ophthalmic, oral, osmotic, parenteral, rectal, sublingual, topical, transdermal, or vaginal. Administering can also be performed, for example, once, a plurality of times, and/or over one or more extended periods and can be a therapeutically effective dose or a subtherapeutic dose.

The term "AUC" (area under the curve) refers to a total amount of drug absorbed or exposed to a subject. Generally, AUC may be obtained from mathematical method in a plot of drug concentration in the subject over time until the concentration is negligible. The term "AUC" (area under the curve) could also refer to partial AUC at specified time intervals (as may be the case with sublingual absorption which would increase AUC at earlier time intervals).

The term "cancer" refers to a broad group of various diseases characterized by the uncontrolled growth of abnormal cells in the body. Unregulated cell division and growth results in the formation of malignant tumors that invade neighboring tissues and can also metastasize to distant parts of the body through the lymphatic system or bloodstream. "Cancer" includes primary, metastatic and recurrent cancers as well as a precancerous condition, *i.e.,* a state of disordered morphology of cells that is associated with an increased risk of cancer. The term "cancer" includes, but is not limited to, the following proliferative diseases: Acute Lymphoblastic Leukemia (ALL), Acute Myeloid Leukemia (AML), Adrenocortical Carcinoms, Childhood cancers, AIDS-Related Cancers, Kaposi Sarcoma, AIDS-Related Lymphoma, Primary CNS Lymphoma, Anal Cancer, Astrocytomas, Atypical Teratoid/Rhabdoid Tumor, Basal Cell Carcinoma, Skin Cancer (Nonmelanoma), Bile Duct Cancer, Bladder Cancer, Bone Cancer, Ewing Sarcoma Family of Tumors, Osteosarcoma and Malignant Fibrous Histiocytoma, Brain Stem Glioma, Atypical Teratoid/Rhabdoid Tumor, Embryonal Tumors, Germ Cell Tumors, Craniopharyngioma, Ependymoma, Breast Cancer, Bronchial Tumors, Burkitt Lymphoma, Non-Hodgkin Lymphoma, Carcinoid Tumor, Gastrointestinal Carcinoma, Cardiac (Heart) Tumors, Primary Lymphoma, Cervical Cancer, Cholangiocarcinoma, Chordoma, Chronic Lymphocytic Leukemia (CLL), Chronic Myelogenous Leukemia (CML), Chronic Myeloproliferative Neoplasms, Colon Cancer, Colorectal Cancer, Craniopharyngioma, Cutaneous T-Cell Lymphoma, Mycosis Fungoides and Sézary Syndrome, Ductal Carcinoma In Situ (DCIS), Embryonal Tumors, Endometrial Cancer, Ependymoma, Esophageal Cancer, Esthesioneuroblastoma, Extracranial Germ Cell Tumor, Extragonadal Germ Cell Tumor, Eye Cancer, Intraocular Melanoma, Retinoblastoma, Fallopian Tube Cancer, Fibrous Histiocytoma of Bone, Malignant, and Osteosarcoma, Gallbladder Cancer, Gastric (Stomach) Cancer, Gastrointestinal Carcinoid Tumor, Gastrointestinal Stromal Tumors (GIST), Germ Cell Tumor, Ovarian, Testicular, Gestational Trophoblastic Disease, Glioma, Hairy Cell Leukemia, Head and Neck Cancer, Hepatocellular (Liver) Cancer, Histiocytosis, Langerhans Cell, Hodgkin Lymphoma, Hypopharyngeal Cancer, Islet Cell Tumors, Pancreatic Neuroendocrine Tumors, Kaposi Sarcoma, Kidney, Renal Cell, Langerhans Cell Histiocytosis, Laryngeal Cancer, Leukemia, Acute Lymphoblastic (ALL), Acute Myeloid (AML), Chronic Lymphocytic (CLL), Chronic Myelogenous (CML), Hairy Cell, Lip and Oral Cavity Cancer, Liver Cancer (Primary), Lung Cancer, Non-Small Cell, Small Cell, Lymphoma, Hodgkin, Non-Hodgkin, Macroglobulinemia, Waldenström, Male Breast Cancer, Melanoma, Merkel Cell Carcinoma, Mesothelioma, Metastatic Squamous Neck Cancer with Occult Primary, Midline Tract Carcinoma Involving NUT Gene, Mouth Cancer, Multiple Endocrine Neoplasia Syndromes, Multiple Myeloma/Plasma Cell Neoplasm, Mycosis Fungoides, Myelodysplastic Syndromes, Myelodysplastic/Myeloproliferative Neoplasms, Myelogenous Leukemia, Chronic (CML), Myeloid Leukemia, Acute (AML) Myeloma, Multiple, Myeloproliferative Neoplasms, Nasal Cavity and Paranasal Sinus Cancer, Nasopharyngeal Cancer, Neuroblastoma, Non-Hodgkin Lymphoma, Non-Small Cell Lung Cancer, Oral Cancer, Oral Cavity Cancer, Lip and Oropharyngeal Cancer, Osteosarcoma and Malignant Fibrous Histiocytoma of Bone, Ovarian Cancer, Low Malignant Potential Tumor, Pancreatic Cancer, Pancreatic Neuroendocrine Tumors (Islet Cell Tumors), Papillomatosis, Paraganglioma, Paranasal Sinus and Nasal Cavity Cancer, Parathyroid Cancer, Penile Cancer, Pharyngeal Cancer, Pheochromocytoma, Pituitary Tumor, Plasma Cell Neoplasm/Multiple Myeloma, Pleuropulmonary Blastoma, Pregnancy and Breast Cancer, Primary CNS Lymphoma, Primary Peritoneal Cancer, Prostate Cancer, Rectal Cancer, Renal Cell (Kidney) Cancer, Renal Pelvis and Ureter, Transitional Cell Cancer, Retinoblastoma, Rhabdomyosarcoma, Salivary Gland Cancer, Rhabdomyosarcoma, Uterine, Small Intestine Cancer, Soft Tissue Sarcoma, Sqamous Cell Carcinoma, Squamous Neck Cancer with Occult Primary, Metastatic, Stomach (Gastric) Cancer, T-Cell Lymphoma, Testicular Cancer, Throat Cancer, Thymoma and Thymic Carcinoma, Thyroid Cancer, Transitional Cell Cancer of the Renal Pelvis and Ureter, Unknown Primary, Ureter and Renal Pelvis, Transitional Cell Cancer, Urethral Cancer, Uterine Cancer, Endometrial, Uterine Sarcoma, Vaginal Cancer, Vulvar Cancer, Waldenström Macroglobulinemia, and Wilms Tumor.

The term "Cₘₐₓ" refers to a maximum concentration of a drug in blood, serum, a specified compartment or test area of a subject between administration of a first dose and administration of a second dose. The term Cₘₐₓ could also refer to dose normalized ratios if specified.

The term "dosing interval" refers to the amount of time that elapses between multiple doses of a pharmaceutical composition disclosed herein being administered to a subject. Dosing interval can thus be indicated as ranges.

The term "disease" means abnormalities in systemic functions resulting from a pathophysiological response to external or internal factors, including disorders, conditions and syndromes, *e*.*g*., a disruption of the disease to the normal or regular functions in the body or a part of the body, a collection or set of signs and symptoms that characterize or suggest a particular disease, or an abnormal state of physical or mental health that interferes with the usual activities or feeling of well-being.

The term "dosing frequency" refers to the frequency of administering doses of a pharmaceutical composition disclosed herein in a given time. Dosing frequency can be indicated as the number of doses per a given time, e.g., once a week or once in two weeks.

The term "effective amount" refers to that amount which is sufficient to effect an intended result. The effective amount will vary depending on the subject and disease state being treated, the severity of the affliction and the manner of administration, and may be determined routinely by one of ordinary skill in the art.

The term "fixed dose" with regard to a pharmaceutical composition refers to two or more different therapeutic agents in a single composition are present in the composition in particular (fixed) ratios with each other. In some embodiments, the fixed dose is based on the weight (*e.g.,* mg) of the therapeutic agents. In some embodiments, the ratio of the therapeutic agents is at least about 1:1, about 1:2, about 1:3, about 1:4, about 1:5, about 1:6, about 1:7, about 1:8, about 1:9, about 1:10, about 1:15, about 1:20, about 1:30, about 1:40, about 1:50, about 1:60, about 1:70, about 1:80, about 1:90, about 1:100, about 1:120, about 1:140, about 1:160, about 1:180, about 1:200, about 200:1, about 180:1, about 160:1, about 140:1, about 120:1, about 100:1, about 90:1, about 80:1, about 70:1, about 60:1, about 50:1, about 40:1, about 30:1, about 20:1, about 15:1, about 10:1, about 9:1, about 8:1, about 7:1, about 6:1, about 5:1, about 4:1, about 3:1, or about 2:1 mg of the first therapeutic agent to mg of the second therapeutic agent.

The terms "in combination with" and "in conjunction with" refer to administration of one treatment modality in addition to another treatment modality. As such, "in combination with" or "in conjunction with" refers to administration of one treatment modality before, during, or after administration of the other treatment modality to the subject.

The term "pharmaceutically acceptable salt" refers to a salt form of one or more of the therapeutic agents described, *e.g*., riluzole, herein which are presented to increase the solubility of the compound in the gastric or gastroenteric juices of the patient's gastrointestinal tract in order to promote dissolution and the bioavailability of the compounds. Pharmaceutically acceptable salts include those derived from pharmaceutically acceptable inorganic or organic bases and acids, where applicable. Suitable salts include those derived from alkali metals such as potassium and sodium, alkaline earth metals such as calcium, magnesium and ammonium salts, among numerous other acids and bases well known in the pharmaceutical art.

The term "prodrug" refers to a precursor of a drug which may be administered in an altered or less active form. The prodrug may be converted into the active drug form in physiological environments by hydrolysis or other metabolic pathways. A discussion of prodrugs is provided in T. Higuchi and V. Stella, Pro-drugs as Novel Delivery Systems (1987) 14 of the A.C.S. Symposium Series*,* and in Bioreversible Carriers in Drug Design, (1987) Edward B. Roche, ed., American Pharmaceutical Association and Pergamon Press.

The term "sublingual administration" refers to a route of administrating a chemical agent or a drug by placing thereof under a tongue of a subject.

The terms "subject" and "patient" refer any human or nonhuman animal. The term "nonhuman animal" includes, but is not limited to, vertebrates such as nonhuman primates, sheep, dogs, and rodents such as mice, rats and guinea pigs. In some embodiments, the subject is a human. The terms, "subject" and "patient" are used interchangeably herein.

The term, "subtherapeutic dose" refers a dose of a therapeutic agent that is lower than the usual or typical dose of the therapeutic agent when administered alone for the treatment of a disease (*e*.*g*., cancer).

The terms "therapeutically effective amount", "therapeutically effective dosage" and "therapeutically effective dose" of an agent (also sometimes referred to herein as a "drug") refers to any amount of the agent that, when used alone or in combination with another agent, protects a subject against the onset of a disease or promotes disease regression evidenced by a decrease in severity of disease symptoms, an increase in frequency and duration of disease symptom-free periods, or a prevention of impairment or disability due to the disease affliction. The ability of an agent to promote disease regression can be evaluated using a variety of methods known to the skilled practitioner, such as in human subjects during clinical trials, in animal model systems predictive of efficacy in humans, or by assaying the activity of the agent in in vitro assays. In certain embodiments, the therapeutically effective amount prevents the development or recurrence of the cancer entirely. "Inhibiting" the development or recurrence of a cancer means either lessening the likelihood of the cancer's development or recurrence, or preventing the development or recurrence of the disease entirely.

The term "Tₘₐₓ" refers to a time or period after administration of a drug when the maximum concentration (Cₘₐₓ) is reached in blood, serum, a specified compartment or test area of a subject.

The term "treatment" refers to any treatment of a condition or disease in a subject and may include: (i) preventing the disease or condition from occurring in the subject which may be predisposed to the disease but has not yet been diagnosed as having it; (ii) inhibiting the disease or condition, *i.e.,* arresting its development; relieving the disease or condition, *i.e.,* causing regression of the condition; or (iii) ameliorating or relieving the conditions caused by the disease, *i.e.,* symptoms of the disease. Treatment could be used in combination with other standard therapies or alone. Treatment or "therapy" of a subject also includes any type of intervention or process performed on, or the administration of an agent to, the subject with the objective of reversing, alleviating, ameliorating, inhibiting, slowing down or preventing the onset, progression, development, severity or recurrence of a symptom, complication or condition, or biochemical indicia associated with a disease.

The term "weight-based dose" refers to a dose that is administered to a patient is calculated based on the weight of the patient. For example, when a patient with 60 kg body weight requires 3 mg/kg of a therapeutic agent, one can administer the appropriate amounts of the therapeutic agent (*i.e.,* 180 mg).

Actual dosage levels of the active ingredient or ingredients in the pharmaceutical compositions of the present invention can be varied so as to obtain an amount of the active ingredient which is effective to achieve the desired therapeutic response for a particular patient, composition, and mode of administration, without being unduly toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

Riluzole is currently available in the market as RILUTEK^{®} (riluzole), which is available from Sanofi-Aventis, Bridgewater, NJ and has the structure shown below.

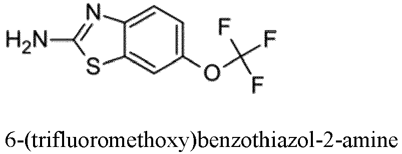

Riluzole, as used in accordance with the present invention, may be present as isotopically labeled forms of compounds detailed herein. Isotopically labeled compounds have structures depicted by the formulas given herein except that one or more atoms are replaced by an atom having a selected atomic mass or mass number. Examples of isotopes that can be incorporated into compounds of the disclosure include isotopes of hydrogen, carbon, nitrogen, oxygen, phosphorous, fluorine and chlorine, such as, but not limited to ²H (deuterium, D), ³H (tritium), ¹³C, ¹⁴C, ¹⁵N, ¹⁸F, ³¹P, ³²P, ³⁵S, ³⁶Cl, and ¹²⁹I. Various isotopically labeled compounds of the present disclosure, for example those into which radioactive isotopes such as ³H, ¹³C and ¹⁴C are incorporated, are provided. Such isotopically labeled compounds may be useful in metabolic studies, reaction kinetic studies, detection or imaging techniques, such as positron emission tomography (PET) or single-photon emission computed tomography (SPECT) including drug or substrate tissue distribution assays or in radioactive treatment of subjects (*e*.*g*., humans). Also provided for isotopically labeled compounds described herein are any pharmaceutically acceptable salts, or hydrates, as the case may be.

In some variations, the compounds disclosed herein may be varied such that from 1 to "n" hydrogens attached to a carbon atom is/are replaced by deuterium, in which "n" is the number of hydrogens in the molecule. Such compounds may exhibit increased resistance to metabolism and are thus useful for increasing the half-life of the compound when administered to a subject. See, *e*.*g*., Foster, "Deuterium Isotope Effects in Studies of Drug Metabolism", Trends Pharmacol. Sci. 5(12):524-527 (1984). Such compounds are synthesized by means well-known in the art, for example by employing starting materials in which one or more hydrogens have been replaced by deuterium.

Deuterium labeled or substituted therapeutic compounds of the disclosure may have improved drug metabolism and pharmacokinetics (DMPK) properties, relating to absorption, distribution, metabolism and excretion (ADME). Substitution with heavier isotopes such as deuterium may afford certain therapeutic advantages resulting from greater metabolic stability, for example increased *in vivo* half-life, reduced dosage requirements and/or an improvement in therapeutic index. An ¹⁸F labeled compound may be useful for PET or SPECT studies. Isotopically labeled compounds of this disclosure can generally be prepared by carrying out the procedures known to those skilled in the art by substituting a readily available isotopically labeled reagent for a non-isotopically labeled reagent. It is understood that deuterium in this context is regarded as a substituent in the compounds provided herein.

The concentration of such a heavier isotope, specifically deuterium, may be defined by an isotopic enrichment factor. In the compounds of this disclosure any atom not specifically designated as a particular isotope is meant to represent any stable isotope of that atom. Unless otherwise stated, when a position is designated specifically as "H" or "hydrogen", the position is understood to have hydrogen at its natural abundance isotopic composition.

The term "riluzole" also refers to all prodrugs, enantiomers, or derivatives and its pharmaceutically acceptable salts, except as otherwise noted. The term "riluzole prodrug" refers to a compound which is a derivative from riluzole with modification therein. A riluzole prodrug may also refer to a compound that is metabolized into an active form of riluzole by the body.

Certain preferred riluzole prodrugs have the structure: 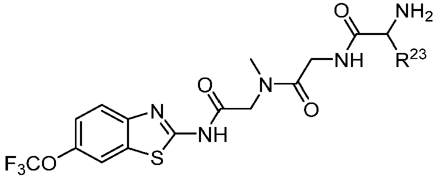 including enantiomers, diastereomers, hydrates, solvates, pharmaceutically acceptable salts, and complexes thereof, wherein:
R₂₃ is selected from the group consisting H, CH₃, CH₂CH₃, CH₂CH₂CH₃, CH₂CCH, CH(CH₃)₂, CH₂CH(CH₃)₂, CH(CH₃)CH₂CH₃, CH₂OH, CH₂OCH₂Ph, CH₂CH₂OCH₂Ph, CH(OH)CH₃, CH₂Ph, CH₂(cyclohexyl), CH₂(4-OH-Ph), (CH₂)₄NH₂, (CH₂)₃NHC(NH₂)NH, CH₂(3-indole), CH₂(5-imidazole), CH₂CO₂H, CH₂CH₂CO₂H, CH₂CONH₂, and CH₂CH₂CONH₂.

One especially preferred prodrug of riluzole is troriluzole (also known as trigriluzole), which has the following formula:

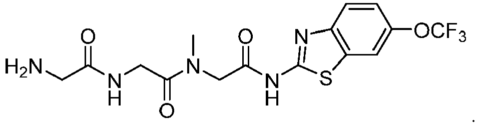

Prodrugs of riluzole are described, for example, in United States Patent Application Serial No. 14/385,551, United States Patent Application Serial No. 14/410,647, PCT Application Serial No. PCT/US2016/019773 and PCT Application Serial No.PCT/US2016/019787. Sublingual formulations of riluzole that provide stability and excellent properties are described in PCT Application Serial No. PCT/US2015/061106 and PCT Application Serial No. PCT/US2015/061114.

The dose of riluzole suitable for use in accordance with the present invention depends on a variety of factors, including, for example, the disease or disorder to be treated, the subject to be treated inclusive of the age, sex, weight and general health condition thereof. In this regard, precise amounts of the agent(s) for administration will depend on the judgment of the practitioner. In determining the effective amount of riluzole to be administered in the treatment or reducing of the conditions associated with the symptoms and disorders, the physician may evaluate clinical factors including symptoms severity or progression of the disorder. The effective amount of the treatment will vary depending on the subject and disease state being treated, the severity of the affliction and the manner of administration, and may be determined routinely by one of ordinary skill in the art. Dosages of riluzole include, for example, for treating a disease or symptoms may be at or below about 400 mg/day, at or below about 300 mg/day, at or below about 150 mg/day, at or below about 120 mg/day, at or below about 80 mg/day, at or below about 40 mg/day, at or below about 20 mg/day, at or below about 10 mg/day, at or below about 5 mg/day, or at or below about 1 mg/day.

The pharmaceutical compositions of the present invention comprising riluzole typically also include other pharmaceutically acceptable carriers and/or excipients such as binders, lubricants, diluents, coatings, disintegrants, barrier layer components, glidants, coloring agents, solubility enhancers, gelling agents, fillers, proteins, co-factors, emulsifiers, solubilizing agents, suspending agents and mixtures thereof. A skilled artisan in the art would know what other pharmaceutically acceptable carriers and/or excipients could be included in the formulations according to the invention. The choice of excipients would depend on the characteristics of the compositions and on the nature of other pharmacologically active compounds in the formulation. Appropriate excipients are known to those skilled in the art (see Handbook of Pharmaceutical Excipients, fifth edition, 2005 edited by Rowe et al., McGraw Hill).

Examples of pharmaceutically acceptable carriers that may be used in preparing the pharmaceutical compositions of the present invention may include, but are not limited to, fillers such as sugars, including lactose, sucrose, mannitol, or sorbitol; cellulose preparations such as maize starch, wheat starch, rice starch, potato starch, gelatin, gum tragacanth, methyl cellulose, hydroxypropyl methyl-cellulose, sodium carboxymethylcellulose, polyvinyl-pyrrolidone (PVP), talc, calcium sulphate, vegetable oils, synthetic oils, polyols, alginic acid, phosphate buffered solutions, emulsifiers, isotonic saline, pyrogen-free water and combinations thereof. If desired, disintegrating agents may be combined as well, and exemplary disintegrating agents may be, but not limited to, cross-linked polyvinyl pyrrolidone, agar, or alginic acid or a salt thereof such as sodium alginate. In an aspect of the invention, the flavoring agent is selected from mint, peppermint, berries, cherries, menthol and sodium chloride flavoring agents, and combinations thereof. In an aspect of the invention, the sweetener is selected from sugar, sucralose, aspartame, acesulfame, neotame, and combinations thereof.

According to the invention, the pharmaceutical compositions containing riluzole are to be administered sublingually. PCT Application No. PCT/US2015/061106 and PCT Application No. PCT/US2015/061114 describe a sublingual formulation of riluzole. When riluzole is prepared as a sublingual formulation, the sublingually administered chemical agent or the drug can diffuse into capillaries through mucous membrane under the tongue, and then enter venous circulation of the subject. As such, sublingual administration may have advantages over oral administration as a conventional tablet by allowing for direct or faster entry to venous circulation, without risks of degradation in gastrointestinal tract, alteration by drug metabolism in liver and the like. Alternatively, the sublingual formulations of the present invention containing riluzole may also be administered such that they are permitted to dissolve on the top of the tongue.

A sublingual formulation useful in the present invention comprises an effective amount of riluzole or pharmaceutically acceptable salts, solvates, anomers, enantiomers, hydrates or prodrugs thereof. The formulation provides sufficient solubility for riluzole to be incorporated into the sublingual formulation and sublingually delivered. The formulation is preferably presented as an oral disintegrating tablet (ODT) of riluzole. In general, the excipients, including mannitol and gelatin, are blended, solubilized with water and deaerated before being mixed with the active pharmaceutical ingredient (API), riluzole, which has been milled separately. Particle size of the API (D₅₀) is less preferably than about 2 microns. The mixture is lyophilized by flash freezing and then freeze-dried. The effective amount of riluzole for the sublingual formulation useful in the present invention to achieve a therapeutically effective dose may be less than that of orally administered agent. Moreover, effective dose of the sublingual formulation of riluzole may be about 1 to 95 %, preferably 50 to 90%, more preferably 70 to 85% and most preferably about 80% of that of the orally administered agent in a conventional tablet, e.g., RILUTEK. For example, an ODT formulation of the present invention may contain about 40 mg of riluzole and have bioequivalence to a 50 mg tablet of RILUTEK.

In one aspect of the invention the pharmaceutical compositions are prepared in oral solid molded fast-dispersing dosage form, such as described in U.S. Patent No. 9,192,580, issued November 24, 2015.

The phrase "fast-dispersing dosage form" refers to compositions which disintegrate or disperse within 1 to 60 seconds, preferably 1 to 30 seconds, more preferably 1 to 10 seconds and particularly 2 to 8 seconds, after being placed in contact with a fluid. The fluid is preferably that found in the oral cavity, *i*.*e*., saliva, as with oral administration. In accordance with the present invention, an ODT is a fast-dispersing dosage form.

In a preferred embodiment, the compositions of the invention are solid fast-dispersing dosage forms comprising a solid network of the active ingredient, rimegepant, and a water-soluble or water-dispersible carrier containing fish gelatin. Accordingly, the carrier is inert towards the active ingredient. The network is obtained by subliming solvent from a composition in the solid state, the composition comprising the active ingredient and a solution of the carrier in the solvent. The dosage forms according to the invention can be prepared according to the process disclosed in Gregory et al., U.K. Patent No. 1,548,022 using fish gelatin as the carrier. Accordingly, an initial composition (or admixture) comprising the active ingredient and a solution of the fish gelatin carrier in a solvent is prepared followed by sublimation. The sublimation is preferably carried out by freeze drying the composition. The composition can be contained in a mold during the freeze-drying process to produce a solid form in any desired shape. The mold can be cooled using liquid nitrogen or solid carbon dioxide in a preliminary step prior to the deposition of the composition therein. After freezing the mold and composition, they are next subjected to reduced pressure and, if desired, controlled application of heat to aid in sublimation of solvent. The reduced pressure applied in the process can be below about 4 mm Hg, preferably below about 0.3 mm Hg. The freeze-dried compositions can then be removed from the mold if desired or stored therein until later use.

When the process is used with active ingredients and fish gelatin as the carrier, a solid fast-dispersing dosage form is produced having the advantages associated with the use of fish gelatin described herein. Generally, fish gelatin is categorized as being from cold water and warm water fish sources and as being of the gelling or non-gelling variety. The non-gelling variety of fish gelatin, in comparison to gelling fish gelatin and bovine gelatin, contains lower proline and hydroxyproline amino acid content, which are known to be associated with cross-linking properties and gelling ability. Non-gelling fish gelatin can remain at solution concentrations of up to about 40% as well as in temperatures as low as 20° C. In one aspect of the invention, the fish gelatin used in accordance with the invention is preferably obtained from cold water fish sources and is the non-gelling type of fish gelatin. More preferably, in one aspect of the invention, the non-hydrolyzed form of non-gelling fish gelatin is used. In an alternative embodiment, spray-dried non-hydrolyzed non-gelling fish gelatin can be used. Fish gelatins suitable for use in the invention are commercially available.

The compositions according to the invention can also contain, in addition to the active ingredient arid fish gelatin carrier, other matrix forming agents and secondary components. Matrix forming agents suitable for use in the present invention include materials derived from animal or vegetable proteins, such as other gelatins, dextrins and soy, wheat and psyllium seed proteins; gums such as acacia, guar, agar, and 10 xanthan; polysaccharides; alginates; carboxymethylcelluloses; carrageenans; dextrans; pectins; synthetic polymers such as polyvinylpyrrolidone; and polypeptide/protein or polysaccharide complexes such as gelatin-acacia complexes.

Other materials which may also be incorporated into the fast-dissolving compositions of the present invention include sugars such as mannitol, dextrose, lactose, galactose, and trehalose; cyclic sugars such as cyclodextrin; inorganic salts such as sodium phosphate, sodium chloride and aluminum silicates; and amino acids having from 2 to 12 carbon atoms such as glycine, L-alanine, L-aspartic acid, L-glutamic acid, L- hydroxyproline, L-isoleucine, L-leucine and L-phenylalanine. One or more matrix forming agents may be incorporated into the solution or suspension prior to solidification (freezing). The matrix forming agent may be present in addition to a surfactant or to the exclusion of a surfactant. In addition to forming the matrix, the matrix forming agent may aid in maintaining the dispersion of any active ingredient within the solution of suspension. This is especially helpful in the case of active agents that are not sufficiently soluble in water and must, therefore, be suspended rather than dissolved. Secondary components such as preservatives, antioxidants, surfactants, viscosity enhancers, coloring agents, flavoring agents, pH modifiers, sweeteners or taste-masking agents may also be incorporated into the fast-dissolving compositions. Suitable coloring agents include red, black and yellow iron oxides and FD & C dyes such as FD&C Blue No. 2 and FD&C Red No. 40 available from Ellis & Everard. Suitable flavoring agents include mint, raspberry, licorice, orange, lemon, grapefruit, caramel, vanilla, cherry and grape flavors and combinations of these. Suitable pH modifiers include the edible acids and bases, such as citric acid, tartaric acid, phosphoric acid, hydrochloric acid, maleic acid and sodium hydroxide. Suitable sweeteners include, for example, sucralose, aspartame, acesulfame K and thaumatin. Suitable taste-masking agents include, for example, sodium bicarbonate, ion exchange resins, cyclodextrin inclusion compounds, adsorbates or microencapsulated actives.

In a preferred aspect of the invention, the fast-dissolving compositions comprises from about 50-70 percent by weight (weight%) riluzole, about 10-30 weight% fish gelatin, about 10-20 weight% of one or more fillers, and 0.1-5.0 weight% of one or more flavorants.

The clinical or therapeutic effect of the riluzole sublingually formulated may have an improved pharmacokinetic profile for the pharmaceutical agent as measured by standard testing parameters. When the riluzole is administered sublingually, one or more of the Tₘₐₓ, Cₘₐₓ and AUC of the drug may be improved compared to the same dose of the orally administered version of the same compound. For example, the sublingual formulation of the riluzole may have a greater Cₘₐₓ than the orally administered riluzole to provide a therapeutically beneficial effect. The sublingual formulation of the riluzole may have an earlier or lesser Tₘₐₓ than the orally administered riluzole to provide a therapeutically beneficial effect and in some instances, a more rapid therapeutic effect. Alternatively, the sublingual formulation of the riluzole may have a greater AUC per milligram of the agent than the orally administered riluzole.

Identifying the subject in need of such treatment can be in the judgment of the subject or a health care professional and can be subjective (*e.g.,* opinion) or objective (*e.g.,* measurable by a test or diagnostic method). The identified subject may be an animal or human in need thereof, particularly a human. Such treatment will be suitably administered to subjects, particularly humans, suffering from the disease.

The therapeutic effect of the pharmaceutical compositions of the present invention may be evident to occur within about a few minutes to about an hour after administration thereof. In particular, the therapeutic effect may begin within about 1 minute, within about 2 minutes, within about 3 minutes, within about 4 minutes, within about 5 minutes, within about 6 minutes, within about 7 minutes, within about 8 minutes, within about 9 minutes, within about 10 minutes, within about 11 minutes, within about 12 minutes, within about 13 minutes, within about 14 minutes, within about 15 minutes, within about 16 minutes, within about 17 minutes, within about 18 minutes, within about 20 minutes, within about 60 minutes, or within about 90 minutes after administration. However, long term cure or amelioration of the disease may not occur for weeks or months after administration.

The effects on the symptoms may be maintained for about 1 hour, for about 2 hours, for about 3 hours, for about 4 hours, for about 5 hours, for about 6 hours m for about 7 hours, for about 8 hours, for about 9 hours, for about 10 hours, for about 12 hours, for about 14 hours, for about 16 hours, for about 18 hours, for about 20 hours, for about 22 hours, for about 24 hours, for about 2 days, or for about 3 days or more after administration thereof. Hopefully, once the long-term effects on the disease state is achieved, the disease, and the symptoms, will be eliminated permanently.

The diseases which may be treated in accordance with the present invention include any diseases in which the administration of riluzole may have a therapeutic or subtherapeutic effect. For example, the disease may be a neuropsychiatric disorder or symptom. In particular, the neuropsychiatric disorder may be anxiety disorders, generalized anxiety disorder, panic disorder, social anxiety, mood disorders, cognitive disorders, schizophrenia, dementia, agitation, apathy, anxiety, psychoses, post-traumatic stress disorders, irritability, disinhibition, learning disorders, memory loss, personality disorders, bipolar disorders, obsessive-compulsive disorders, autism, Rett syndrome, eating disorders, conduct disorders in DSM-5 and or combinations thereof. The disease state may also include neurodegenerative disorders, pain disorders, ALS, cerebellar ataxia, other ataxia, Huntington's disease, Parkinson's disease, supranuclear palsy, frontotemporal dementia, frontotemporal lobar degeneration, delirium, Alzheimer's disease, mild cognitive impairment, mild cognitive impairment due to Alzheimer's disease, drug addiction, tinnitus, and mental retardation.

In addition, the neuropsychiatric symptom may be anxiety, depression, stress, fatigue, feelings of panic, fear, uneasiness, problems in sleeping, cold or sweaty hands and/or feet, mood liability, mania, impaired concentration or attention, cognitive problems, obsessions, compulsions, repetitive behaviors, aggression, social phobias or impairments, stage fright, shortness of breath, heart palpitations, an inability to be still and calm, dry mouth, numbness or tingling in the hands or feet, nausea, muscle tension, dizziness apathy, elation, disinhibition, irritability, wandering, irritable bowel, belly pain, belly discomfort, diarrhea, change in bowel habits, abdominal bloating, abdominal gas, abdominal bloating, constipation or combinations thereof.

In some embodiments, a method may comprise administering to a subject one or more additional agent(s) simultaneously or sequentially with the riluzole. The selection of the additional agents to be administered in combination with riluzole are dependent, among other things, on the disease being treated, the selection of which can be made by one of ordinary skill in the art, *e*.*g*., a physician.

In one aspect, the invention also provides kits for use in the instant methods. Kits can include one or more containers comprising a pharmaceutical composition described herein and instructions for use in accordance with any of the methods described herein. Generally, these instructions comprise a description of administration of the pharmaceutical composition to treat, ameliorate or prevent a disease, *e.g.,* SAD, according to any of the methods described herein. The kit may, for example, comprise a description of selecting an individual suitable for treatment based on identifying whether that individual has SAD. The instructions are typically provided in the form of a package insert, or label, in accordance with the requirements of the regulatory having authority over the jurisdiction where the pharmaceutical composition is to be provided to patients.

### EXAMPLES

The following examples illustrate the invention and are not intended to limit the scope of the invention. In some examples, abbreviations are used which are known to those skilled in the art or are readily accessible from the documents cited in the examples.

### EXAMPLE 1. ACUTE ANXIOLYTIC EFFECTS OF RILUZOLE ON SUBJECTS WITH SOCIAL ANXIETY DISORDER

The study is referred to as 1605017768. The study is further described on ClinicalTrials.gov, ClinicalTrials.gov Identifier: NCT03017508. See https://clinicaltrials.gov/ct2/show/NCT03017508?term=NCT03017508&rank=1.

The primary elements of the protocol used in the study are as follows.

### STUDY DESCRIPTION

### Brief Summary:

The goal of the current study is to examine if sublingual riluzole (BHV-0223) can reduce anxiety in people with social anxiety disorder during a public speaking task.

| **Condition or disease** | **Intervention/treatment** | **Phase** |
|---|---|---|
| Social Anxiety Disorder | Drug: BHV-0223 | Phase 2 |
| Performance Anxiety | Drug: Placebo | Phase 3 |

### Detailed Description:

The investigators conducted a double-blind, placebo-controlled crossover study examining the effects of BHV-0223 on public speaking anxiety. Twenty participants with DSM-5 defined social anxiety disorder and clinically significant public speaking anxiety on the Impromptu Speech Task were enrolled in a challenge study. Participants were given BHV-0223 (or placebo) under double-blind crossover conditions 1 hour prior to performing each of 2 impromptu speech tasks. The two study days involving BHV-0223 (or placebo) administration and impromptu speech task were separated by 2 to 10 days to allow for medication washout. There was a final follow-up visit 2 to 10 days later to perform a complete physical exam and do follow-up liver function testing and a Complete Blood Count. The primary outcome was to examine BHV-0223's effects (compared to placebo) on self-rated anxiety during the impromptu speech task. The investigators also collected physiological measures of anxiety, clinician-rated measures of anxiety, and measures of speech performance as secondary outcomes.

### STUDY DESIGN

| Study Type: | Interventional (Clinical Trial) |
|---|---|
| Estimated Enrollment: | 20 participants |
| Allocation: | Randomized |
| Intervention Model: | Crossover Assignment |
| Masking: | Quadruple (Participant, Care Provider, Investigator, Outcomes Assessor) |
| Official Title: | Double-Blind, Placebo-Controlled, Single-Dose Crossover Study Examining the Effects of Sublingual Riluzole (BHV-0223) on Public Speaking in Social Anxiety Disorder |
| Study Start Date: | January 2017 |
| Estimated Primary Completion Date: | December 2018 |
| Estimated Study Completion Date: | October 2019 |

### ARMS AND INTERVENTIONS

| **Arm** | **Intervention/Treatment** |
|---|---|
| Experimental: BHV-0223 (Sublingual Riluzole) | Drug: BHV-0223 |
| Participants were given one dose of BHV-0223 (sublingual riluzole) 35 mg before performing a 10-minute speech task. Participants were then assessed every hour for the next three hours. There were 2 to 10 days of washout period between the randomly assigned arms of the study. | 35 mg of sublingual riluzole before performing an anxiety provoking speech task. Participants were then clinically assessed every hour for 3 hours. |
| | Other Name: sublingual riluzole |
| Placebo Comparator: Placebo | Drug: Placebo |
| Participants will be given one dose of an identical looking sublingual placebo before performing a 10-minute speech task. Participants will then be assessed every hour for the next three hours. There will be 2 to 10 days of washout period between the randomly assigned arms of the study. | a sublingual tablet identical to the active drug will be given before performing an anxiety provoking speech task. Participants will then be clinically assessed every hour for three hours. |

### OUTCOME MEASURES

### Primary Outcome Measures:

1. VAS-anxiety after the impromptu speech task [Time Frame: 10 minutes] visual analogue scale to assess level of anxiety after performing an anxiety provoking speech task.

### ELIGIBILITY CRITERIA

| | |
|---|---|
| Ages Eligible for Study: | 18 Years to 65 Years (Adult, Older Adult) |
| Sexes Eligible for Study: | All |
| Accepts Healthy Volunteers: | No |

### INCLUSION CRITERIA:

1. Male or female (post-menopausal, surgically sterile, or negative pregnancy test at screening and agreement to utilize an established birth control, including complete abstinence, during the testing period) between the age of 18 and 65 years.
2. Meet DSM-5 criteria for social anxiety disorder by structured clinical interview (SCID) and have a LSAS public speaking subscale score >6.
3. Stable psychiatric medications. Participants must have had stable doses of all psychiatric medications for the month prior to treatment and have been on stable doses of SSRI and antidepressants for at least 1 month prior to study enrollment. As needed benzodiazepine use will be permitted as long as subjects refrain from using benzodiazepines for the 48 hours prior to the study.
4. Medically and neurologically healthy on the basis of physical examination, SMAC-20 (including LFT's, TFT's), VDRL, CBC w/ diff, urinalysis, urine toxicology, EKG, and medical history. Individuals with stable medical problems that do not have CNS effects or interfere with medications administered (*e*.*g*., oral hypoglycemics) may be included if their medications have not been adjusted in the month prior to entry.
5. Urine toxicology screen negative for drug of abuse.
6. Able to provide written informed consent according to the Yale Human Investigation Committee (HIC) guidelines.

### EXCLUSION CRITERIA:

1. Positive pregnancy test.
2. Breastfeeding females.
3. History of substance abuse disorder (ETOH, cocaine, opiates, PCP) within the last 6 months or positive urine toxicology on screening (within the previous 6 months).
4. History of pervasive developmental disorder or psychotic disorder by DSM-IV-TR criteria.
5. Presence of dentures, braces, piercings at the time of dosing, or any physical findings in the mouth or tongue that, in the opinion of the Principal Investigator, would be likely to interfere with successful completion of the dosing procedure.
6. Participants with a medical condition that might interfere with the physiological absorption and motility *(i.e.,* gastric bypass, duodenectomy) or gastric bands.
7. Participants with any clinically significant abnormality or abnormal laboratory test results.
8. Participant has a current diagnosis of viral hepatitis (HBsAG or HVC) or a history of liver disease.
9. Participant has significant history of seizure disorder other than a single childhood febrile seizure (*e.g.,* epilepsy).
10. Participant using any drugs known to induce or inhibit CYP 1A2 metabolism (examples of inducers: rifampin, carbamazepine, etc.; examples of inhibitors: fluvoxamine, ciprofloxacin, fluoroquinolones, etc.) within 30 days prior to the first study drug administration.
11. Participants with a history of allergic reactions to riluzole or other related drugs.
12. Participant has a history of anaphylaxis, a documented hypersensitivity reaction, or a clinically important reaction to any drug.
13. Participant has received another investigational drug or device within the 30 days (90 days for biologics) prior to the first dosing or is currently participating in an investigational study involving no drug administration.
14. Participant with clinically significant electrocardiogram (ECG) abnormalities (QTcF >450 msec) or vital sign abnormalities (systolic blood pressure lower than 90 or over 140 mmHg, diastolic blood pressure lower than 50 or over 90 mmHg, or heart rate less than 50 or over 100 bpm) at Screening or Baseline (Day 1).
15. Any reason which, in the opinion of the Principal Investigator, would prevent the participant from being in the study.

### EXAMPLE 2. SUMMARY OF STUDY RESULTS FROM EXAMPLE 1

The study as substantially described in the protocol set forth in Example 1 was conducted.

The primary purpose of the trial was to examine the acute anti-anxiety potential of BHV-0223 as compared to placebo in subjects with social anxiety disorder and public speaking anxiety while performing a 10-minute anxiety-provoking speech task. Twenty-one subjects who met DSM-5 criteria for social anxiety disorder and clinically significant public speaking anxiety on the Impromptu Speech Task were enrolled in a public speaking challenge study. Subjects were treated with BHV-0223 35mg or placebo under double-blind crossover conditions one hour prior to performing each of two impromptu speech tasks, which were separated by two to ten days to allow for medication washout. The trial was powered at 80%, to detect an effect size of 0.58, at an alpha of p=0.10, on the primary endpoint of self-reported anxiety measured on the VAS during the Impromptu Speech Task. Baseline anxiety was measured on the VAS prior to the speaking exercise.

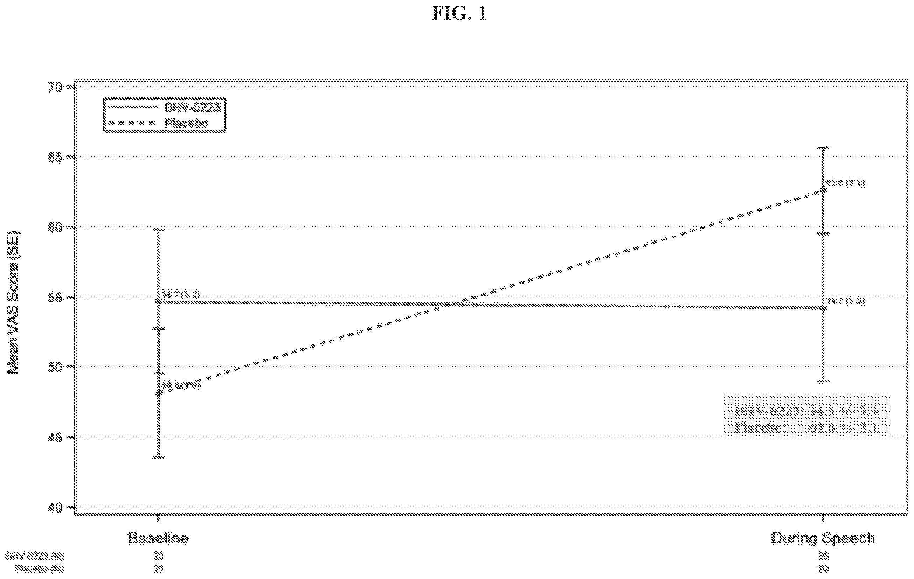

In the pre-specified, primary analysis, BHV-0223 reduced anxiety by 8.3 points relative to placebo on the 100-point Visual Analogue Scale (VAS) (see FIG. 1). The observed reduction in anxiety was significant (p=0.056), relative to the protocol specified level of p= 0.10. A likelihood-based analysis, that analyzed the change in the VAS from the pre-speech baseline, found that BHV-0223 had a 14.4-point advantage relative to placebo (p=0.0259). The trial results are further illustrated in FIG. 1.

BHV-0223 reduced anxiety by 8.3 points relative to placebo on the 100-point Visual Analogue Scale (VAS). The observed reduction in anxiety was significant (p=0.056), relative to the protocol specified level of p= 0.10. A likelihood-based analysis, that analyzed the change in the VAS from the pre-speech baseline, found that BHV-0223 had a 14.4-point advantage relative to placebo (p=0.0259). Preferably in accordance with the present invention, when a patient is tested in accordance with the procedure set forth in Example 1, the administration provides a reduction of at least 12 VAS points, more typically the administration provides a reduction of at least 14 VAS, and even more typically, the administration provides a reduction of from about 10 to 25 VAS points. Preferably, in accordance with the present invention, when a patient is tested in accordance with the procedure set forth in Example 1, the administration provides a mean VAS Score of from about 49 to 60, and more typically the administration provides a mean VAS Score of from about 52 to 58 when tested according to the procedure set forth in Example 1.

### EXAMPLE 3. FURTHER STUDY RESULTS FROM EXAMPLE 1

The study as substantially described in the protocol set forth in Example 1 was conducted.

The study found that BHV-0223 significantly reduced social anxiety relative to placebo.

The study was powered at 80%, to detect an effect size of 0.58, at an alpha of 0.10. The primary endpoint was self-reported anxiety, assessed during a public speaking exercise, and measured on a visual-analog scale (VAS). Baseline anxiety was also measured on a VAS prior to the speaking exercise.

The protocol specified analysis, a repeated measures t-test, was significant at p=0.056 (t=2.03, df--19). This is below the protocol specified alpha level of 0.10. Relative to placebo, BHV-0223 reduced social anxiety by 8.3 point on the VAS (standard error=4.1).

A likelihood-based analysis, that used the change in the VAS from the pre-speech baseline as the dependent variable, found that BHV-0223 reduced social anxiety by 14.4 VAS points relative to placebo (standard error=5.9). This result was significant at p=0.0259 (t=2.41, df=20). The use of a likelihood-based method allowed the inclusion of an additional subject that had partial data. Deletion of that subject from the analysis slightly improved the results, with BHV-0233 reducing social anxiety by 14.9 points relative to placebo (t=2.44, df--19, p=0.0246).

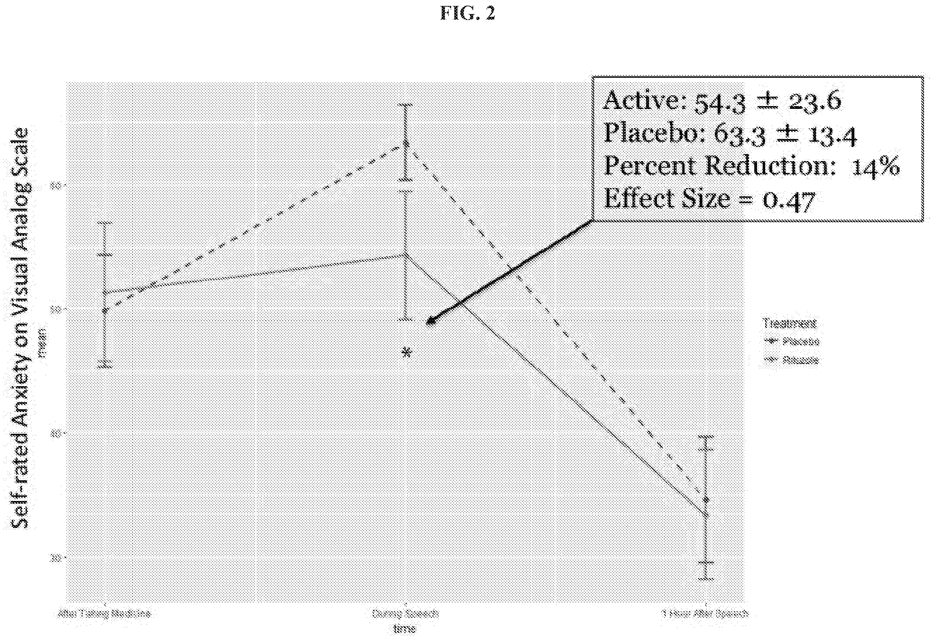

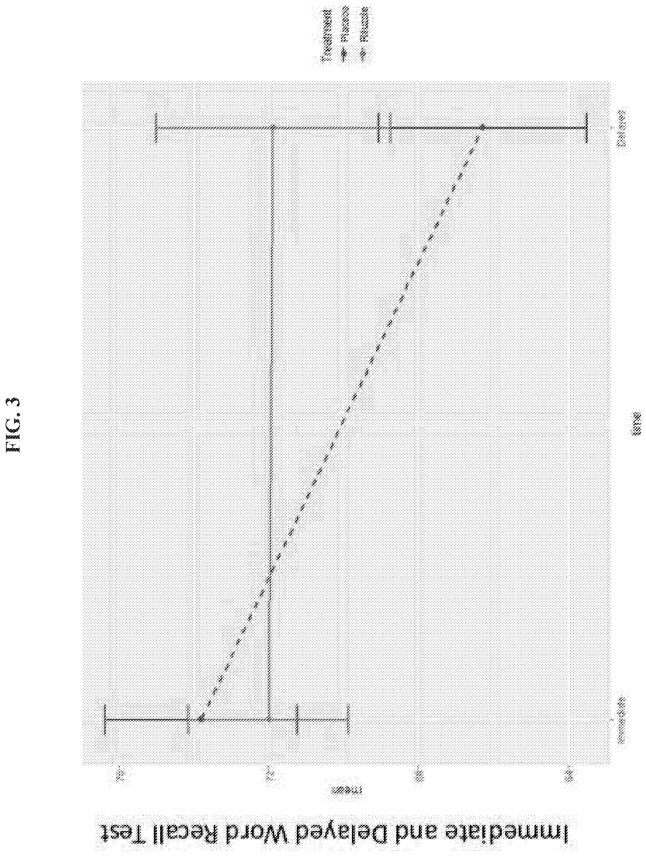

Data obtained in the study is shown in Tables 1, 2, 3 and FIGS. 2 and 3. With respect to the data set shown in Table 3, the following is noted.

| uid | subject id |
|---|---|
| day | study day |
| time | time of rating -1=baseline, 0=study med, 1=speech, 2=1 hour after speech, 3= 2 hour after speech |
| rater | 0=self; 1= inconsistent clinical rater; 2=consistent clinical rater |
| treat | 0=placebo, 1 =riluzole |
| score | VAS score |
| baseline | VAS at baseline of same study day |
| score trim | change in VAS baseline to speech |

**TABLE 1**

| subj id | day | t r e a t | score | baseline | trt | cfb | seq |
|---|---|---|---|---|---|---|---|
| 1 | 1 | 1 | 82.25 | 90 | BHV | -7.75 | BHV_PBO |
| 1 | 2 | 0 | 65.5 | 75 | PBO | -9.5 | BHV_PBO |
| 3 | 1 | 0 | 71.5 | 78 | PBO | -6.5 | PBO_BHV |
| 3 | 2 | 1 | 67.75 | 52 | BHV | 15.75 | PBO_BHV |
| 6 | 1 | 0 | 57.25 | 41 | PBO | 16.25 | PBO_BHV |
| 6 | 2 | 1 | 62 | 60 | BHV | 2 | PBO_BHV |
| 7 | 1 | 1 | 44.5 | 59 | BHV | -14.5 | BHV_PBO |
| 7 | 2 | 0 | 74.5 | 54 | PBO | 20.5 | BHV_PBO |
| 8 | 1 | 0 | 69.25 | 52 | PBO | 17.25 | PBO_BHV |
| 8 | 2 | 1 | 57.75 | 49 | BHV | 8.75 | PBO_BHV |
| 10 | 1 | 1 | 64 | 78 | BHV | -14 | BHV_PBO |
| 10 | 2 | 0 | 67.25 | 73 | PBO | -5.75 | BHV_PBO |
| 11 | 1 | 0 | 50.5 | 55 | PBO | -4.5 | PBO_BHV |
| 11 | 2 | 1 | 54.25 | 67 | BHV | -12.75 | PBO_BHV |
| 12 | 1 | 0 | 68.5 | 75 | PBO | -6.5 | PBO_BHV |
| 12 | 2 | 1 | 30.75 | 37 | BHV | -6.25 | PBO_BHV |
| 14 | 1 | 0 | 55 | 29 | PBO | 26 | PBO_BHV |
| 14 | 2 | 1 | 12.25 | 0 | BHV | 12.25 | PBO_BHV |
| 15 | 2 | 0 | 78.25 | 75 | PBO | 3.25 | BHV_PBO |
| 16 | 1 | 1 | 16.75 | 65 | BHV | -48.25 | BHV_PBO |
| 16 | 2 | 0 | 47 | 25 | PBO | 22 | BHV_PBO |
| 17 | 1 | 0 | 58 | 39 | PBO | 19 | PBO_BHV |
| 17 | 2 | 1 | 23.75 | 33 | BHV | -9.25 | PBO_BHV |
| 18 | 1 | 1 | 64.75 | 57 | BHV | 7.75 | BHV_PBO |
| 18 | 2 | 0 | 68.25 | 62 | PBO | 6.25 | BHV_PBO |
| 20 | 1 | 1 | 29.25 | 35 | BHV | -5.75 | BHV_PBO |
| 20 | 2 | 0 | 27.75 | 22 | PBO | 5.75 | BHV_PBO |
| 21 | 1 | 1 | 92.5 | 52 | BHV | 40.5 | BHV_PBO |
| 21 | 2 | 0 | 75.5 | 41 | PBO | 34.5 | BHV_PBO |
| 22 | 1 | 0 | 77.5 | 0 | PBO | 77.5 | PBO_BHV |
| 22 | 2 | 1 | 67.75 | 79 | BHV | -11.25 | PBO_BHV |
| 24 | 1 | 1 | 79.5 | 82 | BHV | -2.5 | BHV_PBO |
| 24 | 2 | 0 | 83.25 | 62 | PBO | 21.25 | BHV_PBO |
| 27 | 1 | 1 | 68.75 | 40 | BHV | 28.75 | BHV_PBO |
| 27 | 2 | 0 | 60 | 30 | PBO | 30 | BHV_PBO |
| 28 | 1 | 0 | 49.25 | 55 | PBO | -5.75 | PBO_BHV |
| 28 | 2 | 1 | 27.5 | 21 | BHV | 6.5 | PBO_BHV |
| 29 | 1 | 0 | 78.75 | 42 | PBO | 36.75 | PBO_BHV |
| 29 | 2 | 1 | 82.25 | 86 | BHV | -3.75 | PBO_BHV |
| 30 | 1 | 1 | 57 | 52 | BHV | 5 | BHV_PBO |
| 30 | 2 | 0 | 47.5 | 53 | PBO | -5.5 | BHV_PBO |

**TABLE 2**

| xid | uid | day | time | rater | treat | score | baseline | score trim |
|---|---|---|---|---|---|---|---|---|
| 1_1 | 1 | 1 | 1 | 0 | 1 | 82.25 | 90 | -7.75 |
| 1_2 | 1 | 2 | 1 | 0 | 0 | 65.5 | 75 | -9.5 |
| 10_1 | 10 | 1 | 1 | 0 | 1 | 64 | 78 | -14 |
| 10_2 | 10 | 2 | 1 | 0 | 0 | 67.25 | 73 | -5.75 |
| 11_1 | 11 | 1 | 1 | 0 | 0 | 50.5 | 55 | -4.5 |
| 11_2 | 11 | 2 | 1 | 0 | 1 | 54.25 | 67 | -12.75 |
| 12_1 | 12 | 1 | 1 | 0 | 0 | 68.5 | 75 | -6.5 |
| 12_2 | 12 | 2 | 1 | 0 | 1 | 30.75 | 37 | -6.25 |
| 14_1 | 14 | 1 | 1 | 0 | 0 | 55 | 29 | 26 |
| 14_2 | 14 | 2 | 1 | 0 | 1 | 12.25 | 0 | 12.25 |
| 15_2 | 15 | 2 | 1 | 0 | 0 | 78.25 | 75 | 3.25 |
| 16_1 | 16 | 1 | 1 | 0 | 1 | 16.75 | 65 | -48.25 |
| 16_2 | 16 | 2 | 1 | 0 | 0 | 47 | 25 | 22 |
| 17_1 | 17 | 1 | 1 | 0 | 0 | 58 | 39 | 19 |
| 17_2 | 17 | 2 | 1 | 0 | 1 | 23.75 | 33 | -9.25 |
| 18_1 | 18 | 1 | 1 | 0 | 1 | 64.75 | 57 | 7.75 |
| 18_2 | 18 | 2 | 1 | 0 | 0 | 68.25 | 62 | 6.25 |
| 20_1 | 20 | 1 | 1 | 0 | 1 | 29.25 | 35 | -5.75 |
| 20_2 | 20 | 2 | 1 | 0 | 0 | 27.75 | 22 | 5.75 |
| 21_1 | 21 | 1 | 1 | 0 | 1 | 92.5 | 52 | 40.5 |
| 21_2 | 21 | 2 | 1 | 0 | 0 | 75.5 | 41 | 34.5 |
| 22_1 | 22 | 1 | 1 | 0 | 0 | 77.5 | 0 | 77.5 |
| 22_2 | 22 | 2 | 1 | 0 | 1 | 67.75 | 79 | -11.25 |
| 24_1 | 24 | 1 | 1 | 0 | 1 | 79.5 | 82 | -2.5 |
| 24_2 | 24 | 2 | 1 | 0 | 0 | 83.25 | 62 | 21.25 |
| 27_1 | 27 | 1 | 1 | 0 | 1 | 68.75 | 40 | 28.75 |
| 27_2 | 27 | 2 | 1 | 0 | 0 | 60 | 30 | 30 |
| 28_1 | 28 | 1 | 1 | 0 | 0 | 49.25 | 55 | -5.75 |
| 28_2 | 28 | 2 | 1 | 0 | 1 | 27.5 | 21 | 6.5 |
| 29_1 | 29 | 1 | 1 | 0 | 0 | 78.75 | 42 | 36.75 |
| 29_2 | 29 | 2 | 1 | 0 | 1 | 82.25 | 86 | -3.75 |
| 3_1 | 3 | 1 | 1 | 0 | 0 | 71.5 | 78 | -6.5 |
| 3_2 | 3 | 2 | 1 | 0 | 1 | 67.75 | 52 | 15.75 |
| 30_1 | 30 | 1 | 1 | 0 | 1 | 57 | 52 | 5 |
| 30_2 | 30 | 2 | 1 | 0 | 0 | 47.5 | 53 | -5.5 |
| 6_1 | 6 | 1 | 1 | 0 | 0 | 57.25 | 41 | 16.25 |
| 6_2 | 6 | 2 | 1 | 0 | 1 | 62 | 60 | 2 |
| 7_1 | 7 | 1 | 1 | 0 | 1 | 44.5 | 59 | -14.5 |
| 7_2 | 7 | 2 | 1 | 0 | 0 | 74.5 | 54 | 20.5 |
| 8_1 | 8 | 1 | 1 | 0 | 0 | 69.25 | 52 | 17.25 |
| 8_2 | 8 | 2 | 1 | 0 | 1 | 57.75 | 49 | 8.75 |

**TABLE 3**

| subj id | day | trtn | score | baseline | trt | cfb | seq |
|---|---|---|---|---|---|---|---|
| 1 | 1 | 1 | 82.25 | 90 | BHV | -7.75 | BHV_PBO |
| 1 | 2 | 0 | 65.5 | 75 | PBO | -9.5 | BHV_PBO |
| 3 | 1 | 0 | 71.5 | 78 | PBO | -6.5 | PBO_BHV |
| 3 | 2 | 1 | 67.75 | 52 | BHV | 15.75 | PBO_BHV |
| 6 | 1 | 0 | 57.25 | 41 | PBO | 16.25 | PBO_BHV |
| 6 | 2 | 1 | 62 | 60 | BHV | 2 | PBO_BHV |
| 7 | 1 | 1 | 44.5 | 59 | BHV | -14.5 | BHV_PBO |
| 7 | 2 | 0 | 74.5 | 54 | PBO | 20.5 | BHV_PBO |
| 8 | 1 | 0 | 69.25 | 52 | PBO | 17.25 | PBO_BHV |
| 8 | 2 | 1 | 57.75 | 49 | BHV | 8.75 | PBO_BHV |
| 10 | 1 | 1 | 64 | 78 | BHV | -14 | BHV_PBO |
| 10 | 2 | 0 | 67.25 | 73 | PBO | -5.75 | BHV_PBO |
| 11 | 1 | 0 | 50.5 | 55 | PBO | -4.5 | PBO_BHV |
| 11 | 2 | 1 | 54.25 | 67 | BHV | -12.75 | PBO_BHV |
| 12 | 1 | 0 | 68.5 | 75 | PBO | -6.5 | PBO_BHV |
| 12 | 2 | 1 | 30.75 | 37 | BHV | -6.25 | PBO_BHV |
| 14 | 1 | 0 | 55 | 29 | PBO | 26 | PBO_BHV |
| 14 | 2 | 1 | 12.25 | 0 | BHV | 12.25 | PBO_BHV |
| 15 | 2 | 0 | 78.25 | 75 | PBO | 3.25 | BHV_PBO |
| 16 | 1 | 1 | 16.75 | 65 | BHV | -48.25 | BHV_PBO |
| 16 | 2 | 0 | 47 | 25 | PBO | 22 | BHV_PBO |
| 17 | 1 | 0 | 58 | 39 | PBO | 19 | PBO_BHV |
| 17 | 2 | 1 | 23.75 | 33 | BHV | -9.25 | PBO_BHV |
| 18 | 1 | 1 | 64.75 | 57 | BHV | 7.75 | BHV_PBO |
| 18 | 2 | 0 | 68.25 | 62 | PBO | 6.25 | BHV_PBO |
| 20 | 1 | 1 | 29.25 | 35 | BHV | -5.75 | BHV_PBO |
| 20 | 2 | 0 | 27.75 | 22 | PBO | 5.75 | BHV_PBO |
| 21 | 1 | 1 | 92.5 | 52 | BHV | 40.5 | BHV_PBO |
| 21 | 2 | 0 | 75.5 | 41 | PBO | 34.5 | BHV_PBO |
| 22 | 1 | 0 | 77.5 | 0 | PBO | 77.5 | PBO_BHV |
| 22 | 2 | 1 | 67.75 | 79 | BHV | -11.25 | PBO_BHV |
| 24 | 1 | 1 | 79.5 | 82 | BHV | -2.5 | BHV_PBO |
| 24 | 2 | 0 | 83.25 | 62 | PBO | 21.25 | BHV_PBO |
| 27 | 1 | 1 | 68.75 | 40 | BHV | 28.75 | BHV_PBO |
| 27 | 2 | 0 | 60 | 30 | PBO | 30 | BHV_PBO |
| 28 | 1 | 0 | 49.25 | 55 | PBO | -5.75 | PBO_BHV |
| 28 | 2 | 1 | 27.5 | 21 | BHV | 6.5 | PBO_BHV |
| 29 | 1 | 0 | 78.75 | 42 | PBO | 36.75 | PBO_BHV |
| 29 | 2 | 1 | 82.25 | 86 | BHV | -3.75 | PBO_BHV |
| 30 | 1 | 1 | 57 | 52 | BHV | 5 | BHV_PBO |
| 30 | 2 | 0 | 47.5 | 53 | PBO | -5.5 | BHV_PBO |

### EXAMPLE 4. FURTHER STUDY RESULTS FROM EXAMPLE 1

The study as substantially described in the protocol set forth in Example 1 was conducted. Quite surprisingly, as shown in FIG. 3 below, the study demonstrated an improvement in cognitive safety testing performed and showed improvements in delayed recall memory, p<0.05.

Throughout this application, various publications are referenced by author name and date, or by patent number or patent publication number. However, the citation of a reference herein should not be construed as an acknowledgement that such reference is prior art to the present invention.

Those skilled in the art will recognize or be able to ascertain using no more than routine experimentation, numerous equivalents to the specific procedures described herein. Furthermore, it is intended that specific items within lists of items, or subset groups of items within larger groups of items, can be combined with other specific items, subset groups of items or larger groups of items whether or not there is a specific disclosure herein identifying such a combination.

## Claims

1. A sublingual pharmaceutical composition for use in a method of treating an anxiety disorder, the composition comprising riluzole, or a pharmaceutically acceptable salt or prodrug thereof, in the form of an oral solid molded fast-dispersing dosage form;
wherein riluzole prodrugs are according to the following formula: 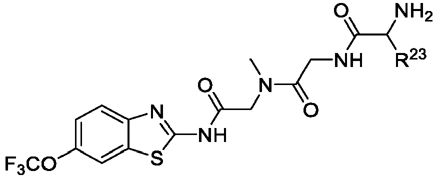 and pharmaceutically acceptable salts thereof, wherein:
R₂₃ is selected from the group consisting H, CH₃, CH₂CH₃, CH₂CH₂CH₃, CH₂CCH, CH(CH₃)₂, CH₂CH(CH₃)₂, CH(CH₃)CH₂CH₃, CH₂OH, CH₂OCH₂Ph, CH₂CH₂OCH₂Ph, CH(OH)CH₃, CH₂Ph, CH₂(cyclohexyl), CH₂(4-OH-Ph), (CH₂)₄NH₂, (CH₂)₃NHC(NH₂)NH, CH₂(3-indole), CH₂(5-imidazole), CH₂CO₂H, CH₂CH₂CO₂H, CH₂CONH₂, and CH₂CH₂CONH₂;
wherein the dosage of riluzole in the oral solid molded fast dispersing dosage form is from 20 to 50 mg, and
wherein the anxiety disorder is social anxiety disorder (SAD).

2. The pharmaceutical composition for use according to claim 1 wherein the dosage of riluzole in the oral solid molded fast dispersing dosage form is about 35 mg.

3. The pharmaceutical composition for use according to claim 1 or 2, wherein the administration provides a reduction of at least 10 Visual Analogue Scale (VAS) points as compared to administration of a placebo.

4. The pharmaceutical composition for use according to any one of the preceding claims wherein the administration provides a reduction of at least 12 VAS points.

5. The pharmaceutical composition for use according to claim 4 wherein the administration provides a reduction of at least 14 VAS points.

6. The pharmaceutical composition for use according to claim 1 wherein the administration provides a reduction of from about 10 to 25 VAS points.

7. The pharmaceutical composition for use according to claim 1 wherein the administration provides a mean VAS Score of from about 49 to 60.

8. The pharmaceutical composition for use according to claim 7 wherein the administration provides a mean VAS Score of from about 52 to 58.

9. The pharmaceutical composition for use according to any one of the preceding claims which provides an enhancement in memory of the patient.

10. The pharmaceutical composition for use according to any one of the preceding claims wherein the oral solid molded fast-dispersing dosage form comprises from about 50-70 weight% riluzole, pharmaceutically acceptable salt or prodrug thereof, about 10-30 weight% fish gelatin, about 10-20 weight% of a filler, and 0.1-5.0 weight% of a flavorant.

11. The pharmaceutical composition for use according to claim 10 wherein the filler is mannitol.

12. The pharmaceutical composition for use according to any one of the preceding claims wherein the composition comprises a riluzole prodrug.

13. The pharmaceutical composition for use according to claim 12 wherein the riluzole prodrug has the following formula:

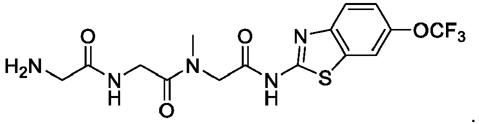

14. A kit for use in a method of treating social anxiety disorder, the kit comprising:
(a) a sublingual pharmaceutical composition according to any one of claims 1 to 13; and
(b) instructions for administering the pharmaceutical composition

## Patentansprüche

1. Sublinguale pharmazeutische Zusammensetzung zur Verwendung in einem Verfahren zur Behandlung einer Angststörung, wobei die Zusammensetzung Riluzol oder ein pharmazeutisch annehmbares Salz oder Solvat davon in Form einer oralen, festen, geformten, rasch dispergierenden Dosierungsform umfasst;
wobei Riluzol-Prodrugs der folgenden Formel: 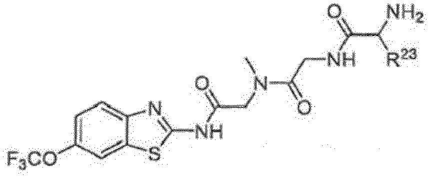 und pharmazeutisch annehmbaren Salzen davon entsprechen, worin:
R₂₃ aus der aus H, CH₃, CH₂CH₃, CH₂CH₂CH₃, CH₂CCH, CH(CH₃)₂, CH₂CH(CH₃)₂, CH(CH₃)CH₂CH₃, CH₂OH, CH₂OCH₂Ph, CH₂CH₂OCH₂Ph, CH(OH)CH₃, CH₂Ph, CH₂(Cyclohexyl), CH₂(4-OH-Ph), (CH₂)₄NH₂, (CH₂)₃NHC(NH₂)NH, CH₂(3-Indol), CH₂(5-Imidazol), CH₂CO₂H, CH₂CH₂CO₂H, CH₂CONH₂ und CH₂CH₂CONH₂ bestehenden Gruppe ausgewählt ist;
wobei die Riluzol-Dosis in der oralen, festen, geformten, rasch dispergierenden Dosierungsform 20 bis 50 mg beträgt und
wobei die Angststörung eine soziale Angststörung (SAD) ist.

2. Pharmazeutische Zusammensetzung zur Verwendung nach Anspruch 1, wobei die Riluzol-Dosis in der oralen, festen, geformten, rasch dispergierenden Dosierungsform etwa 35 mg beträgt.

3. Pharmazeutische Zusammensetzung zur Verwendung nach Anspruch 1 oder 2, wobei die Verabreichung eine Senkung von zumindest 10 Punkten in der visuellen Analogskala (VAS) im Vergleich zur Verabreichung eines Placebos ergibt.

4. Pharmazeutische Zusammensetzung zur Verwendung nach einem der vorangegangenen Ansprüche, wobei die Verabreichung eine Senkung von zumindest 12 VAS-Punkten ergibt.

5. Pharmazeutische Zusammensetzung zur Verwendung nach Anspruch 4, wobei die Verabreichung eine Senkung von zumindest 14 VAS-Punkten ergibt.

6. Pharmazeutische Zusammensetzung zur Verwendung nach Anspruch 1, wobei die Verabreichung eine Senkung von etwa 10 bis 25 VAS-Punkten ergibt.

7. Pharmazeutische Zusammensetzung zur Verwendung nach Anspruch 1, wobei die Verabreichung einen mittleren VAS-Wert von etwa 49 bis 60 ergibt.

8. Pharmazeutische Zusammensetzung zur Verwendung nach Anspruch 7, wobei die Verabreichung einen mittleren VAS-Wert von etwa 52 bis 58 ergibt.

9. Pharmazeutische Zusammensetzung zur Verwendung nach einem der vorangegangenen Ansprüche, die eine Verbesserung des Gedächtnisses des Patienten ergibt.

10. Pharmazeutische Zusammensetzung zur Verwendung nach einem der vorangegangenen Ansprüche, wobei die orale, feste, geformte, rasch dispergierende Dosierungsform etwa 50 bis 70 Gew.-% Riluzol, eines pharmazeutisch annehmbaren Salzes oder eines Pro-drugs davon, etwa 10 bis 30 Gew.-% frische Gelatine, etwa 10 bis 20 Gew.-% eines Füllstoffs und 0,1 bis 5,0 Gew.-% eines Geschmacksstoffs umfasst.

11. Pharmazeutische Zusammensetzung zur Verwendung nach Anspruch 10, wobei der Füllstoff Mannit ist.

12. Pharmazeutische Zusammensetzung zur Verwendung nach einem der vorangegangenen Ansprüche, wobei die Zusammensetzung ein Riluzol-Prodrug umfasst.

13. Pharmazeutische Zusammensetzung zur Verwendung nach Anspruch 12, wobei das Riluzol-Prodrug die folgende Formel aufweist:

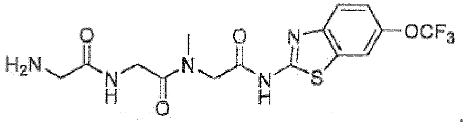

14. Set zur Verwendung in einem Verfahren zur Behandlung einer sozialen Angststörung, wobei das Set Folgendes umfasst:
(a) eine sublinguale pharmazeutische Zusammensetzung nach einem der Ansprüche 1 bis 13; und
(b) Anleitungen zur Verabreichung der pharmazeutischen Zusammensetzung.

## Revendications

1. Composition pharmaceutique sublinguale à utiliser dans un procédé de traitement d'un trouble de l'anxiété, la composition comprenant du riluzole, ou un sel ou un promédicament pharmaceutiquement acceptable de celui-ci, sous la forme d'une forme posologique orale solide moulée à dispersion rapide ;
dans laquelle les promédicaments de riluzole répondent à la formule suivante : 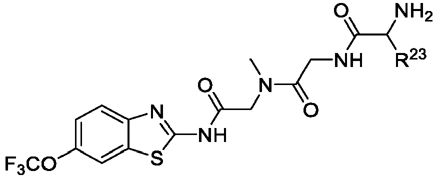 et des sels pharmaceutiquement acceptables de ceux-ci, dans laquelle :
R₂₃ est choisi dans le groupe comprenant H, CH₃, CH₂CH₃, CH₂CH₂CH₃, CH₂CCH, CH(CH₃)₂, CH₂CH(CH₃)₂, CH(CH₃)CH₂CH₃, CH₂OH, CH₂OCH₂Ph, CH₂CH₂OCH₂Ph, CH(OH)CH₃, CH₂Ph, CH₂(cyclohexyle), CH₂(4-OH-Ph), (CH₂)₄NH₂, (CH₂)₃NHC(NH₂)NH, CH₂(3-indole), CH₂(5-imidazole), CH₂CO₂H, CH₂CH₂CO₂H, CH₂CONH₂, et CH₂CH₂CONH₂;
dans laquelle le dosage de riluzole dans la forme posologique orale solide moulée à dispersion rapide est de 20 à 50 mg, et
dans laquelle le trouble de l'anxiété est un trouble de l'anxiété sociale (tas).

2. Composition pharmaceutique à utiliser selon la revendication 1, dans laquelle la dose de riluzole dans la forme posologique orale solide moulée à dispersion rapide est d'environ 35 mg.

3. Composition pharmaceutique à utiliser selon la revendication 1 ou 2, dans laquelle l'administration fournit une réduction d'au moins 10 points d'échelle analogique visuelle (VAS) par rapport à l'administration d'un placebo.

4. Composition pharmaceutique à utiliser selon l'une quelconque des revendications précédentes, dans laquelle l'administration fournit une réduction d'au moins 12 points VAS.

5. Composition pharmaceutique à utiliser selon la revendication 4, dans laquelle l'administration fournit une réduction d'au moins 14 points VAS.

6. Composition pharmaceutique à utiliser selon la revendication 1, dans laquelle l'administration fournit une réduction d'environ 10 à 25 points VAS.

7. Composition pharmaceutique à utiliser selon la revendication 1, dans laquelle l'administration fournit un score VAS moyen d'environ 49 à 60.

8. Composition pharmaceutique à utiliser selon la revendication 7, dans laquelle l'administration fournit un score VAS moyen d'environ 52 à 58.

9. Composition pharmaceutique à utiliser selon l'une quelconque des revendications précédentes, qui améliore la mémoire du patient.

10. Composition pharmaceutique à utiliser selon l'une quelconque des revendications précédentes, dans laquelle la forme posologique orale solide moulée à dispersion rapide comprend environ 50 à 70 % en poids de riluzole, de sel pharmaceutiquement acceptable ou de promédicament de celui-ci, environ 10 à 30 % en poids de gélatine de poisson, environ 10 à 20 % en poids d'une charge et 0,1 à 5,0 % en poids d'un aromatisant.

11. Composition pharmaceutique à utiliser selon la revendication 10, dans laquelle la charge est le mannitol.

12. Composition pharmaceutique à utiliser selon l'une quelconque des revendications précédentes, dans laquelle la composition comprend un promédicament de riluzole.

13. Composition pharmaceutique à utiliser selon la revendication 12, dans laquelle le promédicament de riluzole présente la formule suivante :

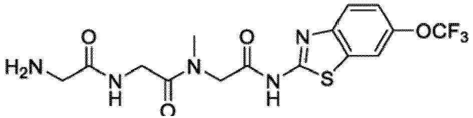

14. Kit à utiliser dans un procédé de traitement d'un trouble de l'anxiété sociale, le kit comprenant :
(a) une composition pharmaceutique sublinguale selon l'une quelconque des revendications 1 à 13 ; et
(b) des instructions pour administrer la composition pharmaceutique.
